# EUROPEAN PATENT SPECIFICATION

(11) **EP 0 918 219 B1**
(45) Date of publication and mention of the grant of the patent: **22.01.2003**
(21) Application number: 98309457.4
(22) Date of filing: 18.11.1998
(51) Int. Cl.: G01N 33/543, G01N 33/552, G01N 33/535

(54) **Sol-gels and their use in water quality assays**
Sol-Gele zur Messung der Wasserqualität
Sol-gels pour la mesure de la qualité de l'eau

(30) Priority: 19.11.1997 EP 97309322
(43) Date of publication of application: 26.05.1999
(73) Proprietor: Randox Laboratories Ltd., Crumlin, Co. Antrim BT29 4QY (GB)
(72) Inventor: Fitzgerald, Stephen Peter, Randox Lab. Ltd., Crumlin, BT29 4QU, Co. Antrim (GB); Lamont, John Victor, Randox Laboratories Ltd., Crumlin, BT29 4QU, Co. Antrim (GB); McConnell, Robert Ivan, Randox Laboratories Ltd., Crumlin, BT29 4QU, Co. Antrim (GB)
(74) Representative: Perry, Robert Edward

(56) References cited:
- EP-A- 0 434 317
- EP-A- 0 439 318
- US-A- 5 200 334
- US-A- 5 608 006
- PIECHOTA I ET AL: "UNTERSUCHUNGEN ZUR SYNTHESE LOESLICHER GLAESER NACH DEM SOL-GEL VERFAHREN UND ZUR KONTROLLIERTEN FREISETZUNG VON FLUORESCEIN-NATRIUM" ACTA PHARMACEUTICA TECHNOLOGICA, vol. 34, 1988, page 275 XP002061193
- BRAUN S ET AL: "DESIGN AND PROPERTIES OF ENZYMES IMMOBILIZED IN SOL-GEL GLASS MATRICES" 24 June 1990 , BIOTECHNOLOGY: BRIDGING RESEARCH AND APPLICATIONS, PAGE(S) 205 - 218 XP002061194 * page 205 - page 218 *

## Description

### Field of the Invention

This invention relates to sol-gels and their use in water quality assays.

### Background of the Invention

Water courses may be contaminated with substances such as sewage, heavy metals, pesticides containing organic residues etc. Such substances act as free radical scavengers. They can therefore be assayed using a light-producing free radical reaction; the light emission is reduced or inhibited to a degree proportional to the amount of contaminant present, when compared to a distilled water control.

One such test, available under the trade name Aquanox, from Randox Laboratories Limited, involves a free radical reaction between a hydrogen acceptor (oxidant) and a hydrogen donor (luminol) in the presence of an enhancer. This reaction is catalysed by horseradish peroxidase (HRP) and results in light emission at a constant rate.

At present, in order for the Aquanox assay to be performed, a vial of freeze-dried signal reagent (containing luminol, enhancer and oxidant) is reconstituted with 5 ml borate buffer (pH 8.5), and then 100 µl of this solution, 20 µl enzyme reagent plus 1 ml water sample are added to a disposable cuvette. The reaction is started by the addition of the enzyme reagent. The separate additions of signal reagent, enzyme and sample to the cuvette have proved difficult for some users when undertaken in the field, especially to less technically skilled personnel.

Sol-gels are known. The sol-gel process involves the mixing of metal alkoxides, e.g. TMOS, i.e. tetramethoxysilane, in solution with water and a catalyst, at room temperature. In this process, a complex series of hydrolysis and polymerisation reactions occurs. The initially fluid solution becomes viscous as polymerisation proceeds, until a gel is formed. The gel is then dried, during which process liquid is expelled, causing substantial volume shrinkage of the gel, leaving a dry porous solid. The pore networks formed in dried gels do not scatter visible radiation and are therefore optically transparent.

Molecules added in the sol-gel process become entrapped in the growing covalent network. For example, Piechota and Sueverkruep, Acta. Pharm. Technol. 34:27s (1988), discloses sol-gels of unspecified composition, containing fluorescein. The marker was released at an essentially constant rate over 5-8 hours, and the rate varied with increasing phosphate content.

EP-A-0439318 discloses a reagent trapped in a sol-gel glass, with the intention that a desired reaction with an analyte should occur within the pores of the network. The Examples disclose TMOS:solvent ratios of approx. 2:3 for sol-gel shapes and 1:8 for thin films.

US-A-5200334 discloses an active biological material encapsulated in a sol-gel. Improved shelf-life is demonstrated.

### Summary of the Invention

The present invention is based on the discovery that, at a suitable TMOS:solvent ratio, sol-gels can be produced that satisfactorily retain a reactant therein, but which can readily be leached out in the presence of water. This is particularly suitable when it is desired to supply reactants to a user in a mixture, but physically separate, until use.

According to the invention, for a combination of first and second reactants that give a signal when mixed in the presence of an analyte in an aqueous liquid sample, a combination comprises the reactants separately contained in sol-gels that release the reactants in the presence of the liquid.

### Description of the Invention

By virtue of the invention, the reactants that are required for use in, say, the Aquanox system can be supplied in dry/solid form, e.g. as a dry mixture of any desired shape, films, pellets or powder. For the purpose of this specification, the term "reactant" is used to describe one or more components. If two or more components are contained in one sol-gel, they should not be mutually reactive.

The reactants that may be used in the present invention are not limited. Examples include organic and inorganic ligands, antibodies, enzymes, oxidising and reducing agents, reaction enhancers, signal-generators and labels. Similarly, the components of the sol-gels are not limited, although TMOS is preferred. The preferred alkoxide:water ratio (by volume) is 2.5:1 to 5:1.

The invention will now be described by way of example only with reference to the components used in the Aquanox system. Thus, the first reactant comprises at least luminol as hydrogen donor (reductant) and the second reactant comprises sodium perborate as hydrogen acceptor (oxidant). The first reactant may additionally comprise horseradish peroxidase, and either reactant may additionally comprise enhancer (p-iodophenol), or either or each of these additional components may be contained in further sol-gels. For use in an Aquanox cuvette, these four components may be provided in dried pellet form; no interaction occurs until the water sample is added. However, once water is added, the gels are such that sufficient of the components quickly leach out of their respective sol-gel pellets, and react together, free in solution.

The following Examples illustrate the invention, or are for the purposes of comparison. More specifically, Examples 1 and 2 (TMOS:water ratio is 5:1 or 5:2) are illustrative; Examples 3 to 5 (TMOS:water ratio is 3:2, 1:1 or 1:2) are comparative.

In the Examples, TMOS is tetramethoxysilane/tetramethyl orthosilicate. Citrate phosphate buffer, pH 6.0, comprises the acid and Na₂HPO₄. Borate buffer, pH 8.5, comprises sodium tetraborate, N-methylisothiazolone and 2-chloroacetamide.

### Preparation of Sol-Gels

1. Sol-gel pellets of total volume 200 µl were prepared in the wells of Nunc clear microtitre plates, by the following method:
   (i) HRP-pellets - into each well was pipetted 100 µl pH 6.0 citrate phosphate buffer which contained the following:
      (a) 1, 1.5 or 2 mg/ml HRP only
      (b) 1, 1.5 or 2 mg/ml HRP plus 1% polyvinyl alcohol (9000-10,000 mw) or 1% polysucrose (400,000 mw).
   (ii) Luminol/*p*-iodophenol pellets - into each well was pipetted 100 µl pH 8.0 borate buffer which contained 12 mM luminol and 1.2 mM *p*-iodophenol.
   (iii) Sodium perborate pellets - into each well was pipetted 100 µl pH 8.0 borate buffer which contained 50 mM or 100 mM sodium perborate.
2. Into each well was pipetted 100 µl TMOS sol containing, per ml, 30 µl 40 mM HCl and one of the following ratios of TMOS:H₂O - 5:1, 5:2, 3:2, 1:1 and 1:2. The TMOS sols were prepared in brown glass vials and the reagents were mixed by vortexing for approximately 30 seconds, until the solution was clear and the initial two layers had disappeared. After vortexing, the vials were cooled to room temperature before the sol was added to the wells.
   Once the 100 µl TMOS sol was added to the wells, the concentration of reagents was therefore half the value initially present in the well, i.e. 0.5, 0.75 or 1 mg/ml HRP or 6 mM luminol/0.6 mM iodophenol or 25 and 50 mM sodium perborate.
3. After gelling occurred (within minutes), the HRP-containing gels were dried at ambient room temperature by placing the microtitre plates in a desiccator containing silica gel, in the dark, until no further weight loss was recorded. Those gels containing the signal reagent components were dried *in vacuo* in the desiccator, at ambient room temperature, in the dark.

### Assay of Sol-Gels

The control Aquanox assay contained 1 ml deionised water, 100 µl reconstituted signal reagent and 20 µl enzyme reagent. The sol-gel pellets were assayed as follows:
(i) 1 x HRP pellet + 1 ml deionised water + 100 µl reconstituted signal reagent.
(ii) 1 x HRP pellet + 1 x luminol/iodophenol pellet + 1 x sodium perborate pellet + 1 ml deionised water.
(iii) 1 x luminol/iodophenol pellet + 1 x sodium perborate pellet + 20 µl enzyme reagent.
(iv) The wash-water from the above pellets + the corresponding liquid enzyme or reconstituted signal reagent. To obtain this wash-water, 1 ml of deionised water was added to a pellet in a cuvette (or 2 pellets if luminol/iodophenol + perborate pellets were used) and the cuvette left for a specific length of time, up to 4 mins. The water was then pipetted off and assayed.

### Results

The accompanying figures are plots of RLU (relative light units) against T (time; sec). These results are representative examples of the assays performed using a mixture of the individual dried sol-gel pellets and current liquid Aquanox components. An Aquanox (liquid constituents) control graph is included, for comparison.

Figure 1 is the Aquanox control graph, obtained using 1 ml deionised water, 100 µl reconstituted signal reagent and 20 µl enzyme reagent.

Figure 2 illustrates results obtained for each of Examples 1 to 5, and the effect of altering the TMOS:H₂O ratio (in the 100 µl TMOS sol added to each well). The 1 ml wash-water from soaking pellets containing 1 mg/ml HRP was assayed with liquid signal reagent for each of the TMOS:water ratios, therefore indicating the amount of HRP leaching. The results are given in Table 1.

**Table 1**

| Example | TMOS:H₂O | 2 min RLU | 4 min RLU | Integrated RLU |
|---|---|---|---|---|
| 1 | 5:1 | 9490 | 9352 | 2251534 |
| 2 | 5:2 | 10977 | >9999 | 2633369 |
| 3 | 3:2 | 142 | 72 | 18976 |
| 4 | 1:1 | 581 | 616 | 137552 |
| 5 | 1:2 | 100 | 88 | 21974 |

Figure 3 again illustrates the effect of altering the TMOS:H₂O ratio (in the 100 µl TMOS sol added to each well). The HRP-pellets (1 mg/ml) were assayed with 100 µl signal reagent. The results are given in Table 2.

**Table 2**

| Example | TMOS:H₂O | 2 min RLU | 4 min RLU | Integrated RLU |
|---|---|---|---|---|
| 1 | 5:1 | 14320 | >9999 | 3451516 |
| | | 14356 | >9999 | 3451516 |
| 2 | 5:2 | 7810 | 7571 | 1856696 |
| | | 8193 | 7901 | 1758114 |
| 3 | 3:2 | 207 | 200 | 44923 |
| | | 420 | 396 | 84010 |
| 4 | 1:2 | 942 | 930 | 212148 |
| | | 451 | 431 | 98963 |
| 5 | 1:1 | 681 | 789 | 159774 |
| | | 365 | 353 | 79096 |

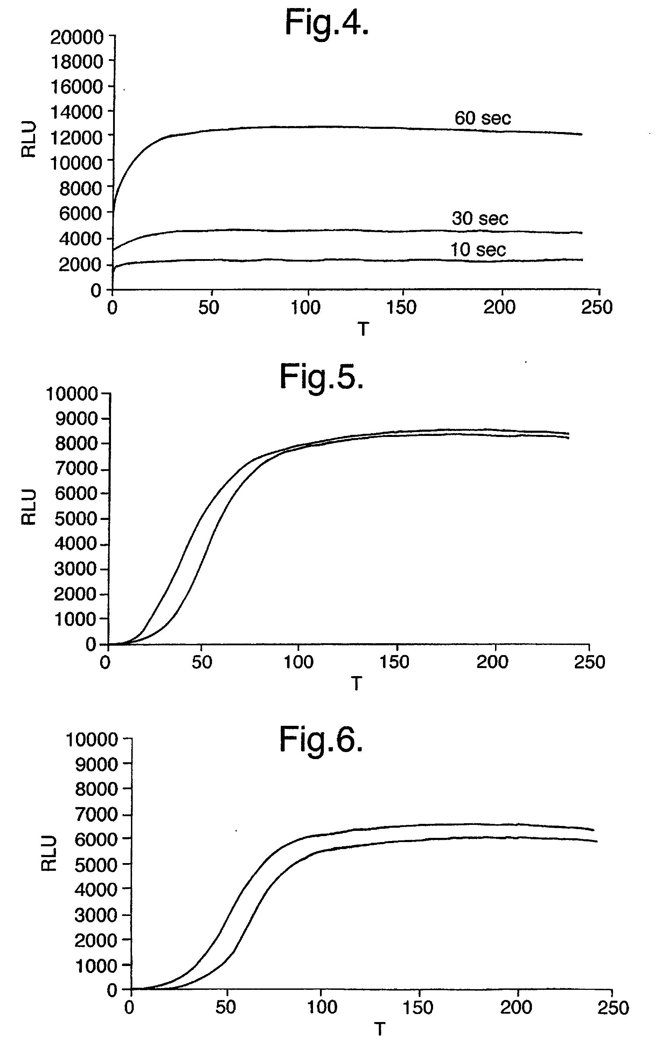

Figure 4 illustrates the activity obtained from timed washings of signal reagent sol-gel pellets (6 mM/0.6 mM luminol/iodophenol + 25 mM sodium perborate). These washing were assayed with 20 µl enzyme reagent. The results are given in Table 3.

**Table 3**

| Wash (sec) | 2 min RLU | 4 min RLU | Integrated RLU |
|---|---|---|---|
| 60 | 12643 | 9999 | 2946658 |
| 30 | 4690 | 4431 | 1083869 |
| 10 | 2764 | 2760 | 639809 |

Figures 5 and 6 illustrate the activity obtained with signal sol-gel pellets assayed with 5 µl enzyme reagent (6/0.6 mM luminol/iodophenol + 25 or 50 mM sodium perborate). The corresponding figures are given in Tables 4 (for 50 mM) and 5 (for 25 mM).

**Table 4**

| 2 min RLU | 4 min RLU | Integrated RLU |
|---|---|---|
| 8235 | 8327 | 1620539 |
| 8115 | 8185 | 1520189 |

**Table 5**

| 2 min RLU | 4 min RLU | Integrated RLU |
|---|---|---|
| 6468 | 6442 | 1220093 |
| 5813 | 5956 | 1051484 |

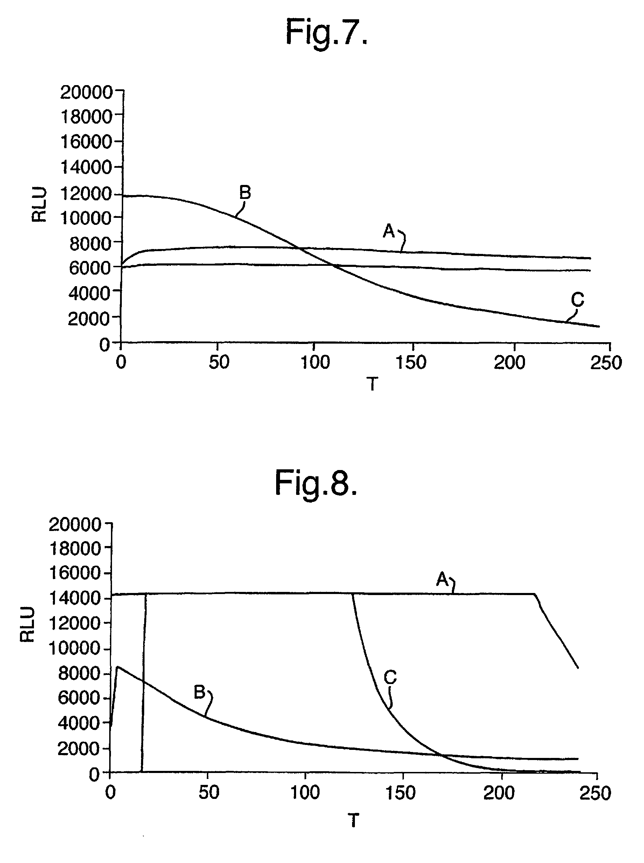

Figures 7 and 8 illustrate the results obtained from HRP-doped pellets containing 0.75 mg/ml and 1 mg/ml, respectively, of the additives polysucrose (mw 400,000) or polyvinyl alcohol (mw 9000-10,000), assayed with liquid signal reagent (A) or signal sol-gel pellets (B). Assays C uses the wash of HRP sol-gel and liquid signal. The corresponding figures are given in Tables 6 (0.75 mg/ml with PVA) and 7 (1 mg/ml with polysucrose).

**Table 6**

| Assay | 2 min RLU | 4 min RLU | Integrated RLU |
|---|---|---|---|
| A | 7521 | 7428 | 1799657 |
| B | 5479 | 2058 | 1513912 |
| C | 6370 | 6410 | 1523811 |

**Table 7**

| Assay | 2 min RLU | 4 min RLU | Integrated RLU |
|---|---|---|---|
| A | 14284 | >9999 | 3369241 |
| B | 2089 | 1054 | 680056 |
| C | 13978 | 252 | 1818469 |

In the case of Assay C, the signal reagent is depleted before 4 min; this observation demonstrates that the HRP is leaching from the sol-gel since the time taken to deplete the signal reagent is longer for the HRP sol-gel + liquid signal than for the wash water from the HRP sol-gel + liquid signal.

The results clearly demonstrate that a primary object of the invention was achieved. The four components of the Aquanox water testing assay (HRP, luminol/*p*-iodophenol and sodium perborate) were successfully encapsulated into TMOS sol-gels and these gels, once dried, were active on the addition of deionised water.

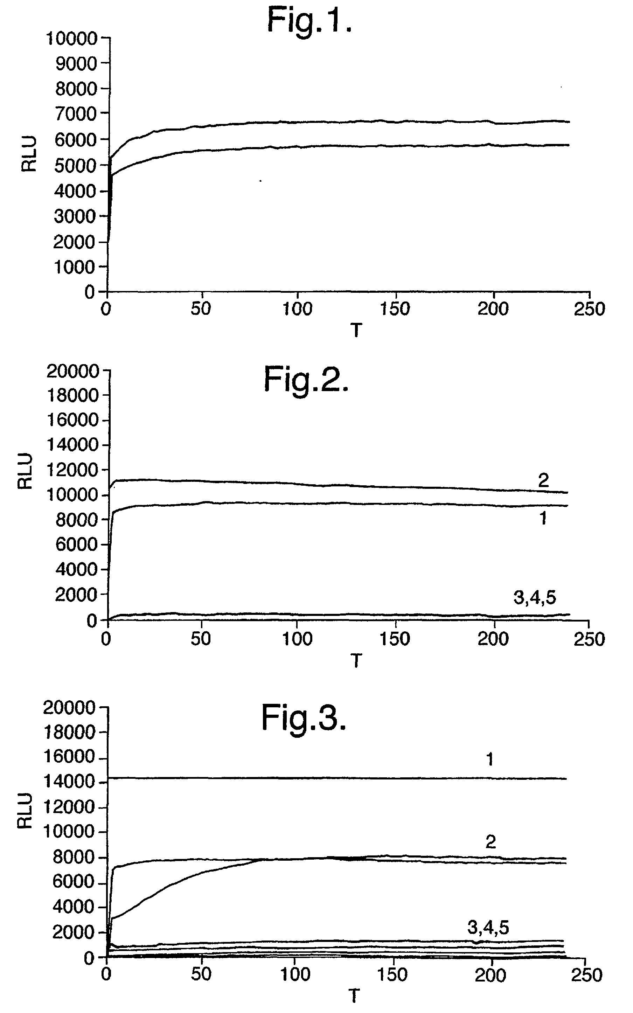

The pellets demonstrated leaching of their enzyme or signal reagent components on addition of water within the 4 minute timescale of the Aquanox assay (see Figs. 2 and 4) at the higher ratios of TMOS:H₂O. The results for the lower ratios, i.e. 3:2, 1:1 and 1:2, were dramatically different, showing very little leaching of the enzyme, and certainly insufficient to perform the Aquanox reaction. This effect was demonstrated whether the wash water of these pellets was assayed with liquid signal (see Fig. 2) or whether the pellets themselves were present in the cuvette (see Fig. 3).

It should be noted that the ratio of TMOS:solvent reported for standard mixtures in EP-A-0439318 was approx. 2:3 for sol-gel shapes, and 1:8 for thin-films; very little leachability was detected in the above results, at such low TMOS:H₂O ratios. In describing the preparation of sol-gel immobilised enzymes, EP-A-0439318 reports that, after water washing of the gel, no significant enzyme activity was detected in the eluates. The method was based on chemical interaction of reagent(s) trapped in sol-gel glass which could interact with diffusible components.

The addition of a high molecular weight component, e.g. above 5,000 mw, enables less HRP to be added to the gels to achieve equivalent RLU values; see Figs. 3 (ratio 5:2 curve at 1 mg/ml) and 7 and 8 (equivalent or higher signals with 0.75 mg/ml or 1 mg/ml). Such additives enable a reduction of up to 50% HRP in the gels, to achieve the required RLU values.

Both HRP and signal components obviously leached out of their respective pellets, as assays of the wash-water fractions demonstrated. Those assays containing both HRP and signal components in sol-gel pellets also demonstrated good activity, presumably based on the interactions of the components once they had leached out of the respective gels (see Figs. 7 and 8). The sol-gels could successfully replace the liquid signal and enzyme reagents in control Aquanox assays (see Figs. 3, 5 and 6), producing RLU values of the magnitude required and also, with further pore size manipulation, the required constant rate of light emission.

These results demonstrate the effectiveness of a solid, ready-to-use chemiluminescent reagent which, once deposited into test cuvettes, requires only one pipetting step, the addition of the water sample, thus simplifying the procedure and offering advantages over the existing system in the field. The cuvettes may be supplied, for use in an existing Aquanox machine, with the sol-gel pellets predispensed.

Improved results may be obtained by further optimisation of the pore size of the gel. Leaching may be enhanced by manipulation of the additives and TMOS ratio. In particular, varying the ratio of components will allow control over the rate of release. Another relevant factor is the size of particles; crushing provides modification in the context of curve B in Fig. 7.

If desired, the four components that are provided separately, in the Examples, may be combined into a reduced number of sol-gels, thus simplifying production. The sol-gels may be produced as thin films which may be layered on to the base/sides of the cuvettes. It will be evident that the solid chemiluminescent reagent is not limited to use in the particular water quality testing system that is illustrated, and that it is applicable to other assay systems employing such a chemiluminescent reaction.

## Claims

1. A composition comprising first and second reactants that combine to give a signal when mixed in the presence of an analyte in an aqueous liquid sample, wherein the reactants are separately contained in sol-gels that release the reactants in the presence of the liquid.

2. A composition according to claim 1, in the form of a dry mixture.

3. A composition according to claim 1 or claim 2, wherein the first and second reactants respectively comprise an oxidant and a reductant, and the reaction provides a detectable signal.

4. A composition according to claim 3, wherein the reductant is luminol, and the composition additionally comprises horseradish peroxidase and an enhancer contained in the same or separate sol-gels.

5. A composition according to any preceding claim, wherein one or each sol-gel is obtainable by reaction of water with, per part by volume thereof, at least 2 parts of tetrametal orthosilicate (TMOS), and drying the resultant gel.

6. A composition according to claim 5, wherein the reaction comprises 2.5 to 5 parts TMOS per part water.

7. A composition according to any preceding claim, wherein the or each sol-gel contains a high molecular weight component.

8. Use of a composition according to any preceding claim, in a water quality assay.

## Patentansprüche

1. Zusammensetzung, umfassend erste und zweite Reaktanten, welche sich vereinigen, um ein Signal zu ergeben, wenn sie in Gegenwart eines Analyten in einer wässerigen Flüssigkeitsprobe gemischt werden, wobei die Reaktanten separat in Sol-Gelen enthalten sind, welche die Reaktanten in Gegenwart der Flüssigkeit freisetzen.

2. Zusammensetzung nach Anspruch 1 in Form einer Trockenmischung,

3. Zusammensetzung nach Anspruch 1 oder Anspruch 2, worin die ersten bzw. zweiten Reaktanten ein Oxidationsmittel und ein Reduktionsmittel umfassen und die Reaktion ein nachweisbares Signal ergibt.

4. Zusammensetzung nach Anspruch 3, worin das Reduktionsmittel Luminol ist und die Zusammensetzung zusätzlich Meerettichperoxidase und einen Verstärker umfaßt, die in denselben oder separaten Sol-Gelen enthalten sind.

5. Zusammensetzung nach irgendeinem der vorhergehenden Ansprüche, worin ein oder jedes Sol-Gel durch Reaktion von Wasser mit, pro Volumenteil davon, mindestens 2 Teilen Tetrametallorthosilicat (TMOS) und Trocknen des resultierenden Gels erhältlich ist.

6. Zusammensetzung nach Anspruch 5, worin die Reaktion 2,5 bis 5 Teile TMOS pro Teil Wasser umfaßt.

7. Zusammensetzung nach irgendeinem der vorhergehenden Ansprüche, worin das oder jedes Sol-Gel eine Komponente mit hohem Molekulargewicht enthält.

8. Verwendung einer Zusammensetzung nach irgendeinem der vorhergehenden Ansprüche in einem Wasserquaiitäts-Assay.

## Revendications

1. Composition comprenant un premier et un second réactifs qui se combinent pour donner un signal lorsqu'ils sont mélangés en présence d'un analyte dans un échantillon liquide aqueux, dans laquelle les réactifs sont contenus séparément dans des sol-gels qui libèrent les réactifs en présence du liquide.

2. Composition selon la revendication 1, sous la forme d'un mélange sec.

3. Composition selon la revendication 1 ou la revendication 2, dans laquelle le premier et le second réactifs comprennent respectivement un oxydant et un réducteur, et la réaction fournit un signal détectable.

4. Composition selon la revendication 3, dans laquelle le réducteur est un luminol, et la composition comprend en outre de la peroxydase de radis noir et un renforçateur contenu dans le même sol-gel ou des sol-gels séparés.

5. Composition selon l'une quelconque des revendications précédentes, dans laquelle un, ou chaque sol-gel peut être obtenu par réaction d'eau avec, pour chaque partie en volume de celle-ci, au moins 2 parties d'orthosilicate tétramétallique (TMOS), et séchage du gel résultant.

6. Composition selon la revendication 5, dans laquelle la réaction comprend 2,5 à 5 parties de TMOS par partie d'eau.

7. Composition selon l'une quelconque des revendications précédentes, dans laquelle le ou chaque sol-gel contient un composant de haut poids moléculaire.

8. Utilisation d'une composition selon l'une quelconque des revendications précédentes, dans un test de qualité de l'eau.
